(19) Europäisches Patentamt
European Patent Office
Office européen des brevets

(11) Numéro de publication: **0 433 267 B1**

(12) # FASCICULE DE BREVET EUROPEEN

(45) Date de publication de fascicule du brevet: **21.09.94**

(51) Int. Cl.[5]: **C07C 57/18**, C07C 51/353, C07C 17/26, C07C 21/22

(21) Numéro de dépôt: **91101995.8**

(22) Date de dépôt: **30.12.87**

(60) Numéro de publication de la demande initiale en application de l'article 76 CBE : **0 279 954**

(54) **Préparation de l'acide éicosatétraynoique.**

(30) Priorité: **31.12.86 FR 8618419**

(43) Date de publication de la demande: **19.06.91 Bulletin 91/25**

(45) Mention de la délivrance du brevet: **21.09.94 Bulletin 94/38**

(84) Etats contractants désignés: **AT BE CH DE ES FR GB GR IT LI LU NL SE**

(56) Documents cités:
**EP-A- 0 209 770**
**US-A- 3 033 884**

**CHEMICAL ABSTRACTS, vol. 106, no. 19, 11 mai 1987, page 634, abrégé no.155871r, Columbus, Ohio, US; Y.Y. BELOSLUDTSEV et al.: "A new synthesis of 5,8,11,14-eicosatetraynoic acid"**

(73) Titulaire: **CENTRE INTERNATIONAL DE RECHERCHES DERMATOLOGIOUES GALDERMA - CIRD GALDERMA Groupement d'Intérêt Economique dit:
Sophia Antipolis
F-06560 Valbonne (FR)**

(72) Inventeur: **Shroot, Braham
18bis Boulevard Albert 1er
F-06600 Antibes (FR)**
Inventeur: **Hensby, Christopher
241, route des Clausonnes
F-06410 Biot (FR)**
Inventeur: **Maignan, Jean
8, Rue Halévy
F-93290 Tremblay les Gonesses (FR)**
Inventeur: **Lang, Gérard
44, Avenue Lacour
F-95210 Saint-Gratien (FR)**
Inventeur: **Restle, Serge
3-10-7 Mejiro,
Toshima-ku
T 171 Tokyo (JP)**
Inventeur: **Colin, Michel
10 Allée Cécile
F-93190 Livry-Gargan (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**CHEMICAL ABSTRACTS, vol. 97, no. 5, 2 août 1982, page 550, abrégé no. 38706a,Columbus, Ohio, US; L. YAKUSHEVA et al.: "Synthesis of cis, cis, cis-8,11,14-eicosatrienic and [3H]dihomo-gamma-linoleic acids and their conversion to prostaglandins E1,F1a, [3H]-E1 and [3H]-F1a"**

(74) Mandataire: **Casalonga, Axel et al**
**BUREAU D.A. CASALONGA - JOSSE**
**Morassistrasse 8**
**D-80469 München (DE)**

## Description

L'acide eicosatétraynoïque-5,8,11,14 est connu an lui-même, notamment par le brevet US-A-3.033.884. Il est utile notamment dans la préparation de ses amides, dont certains pour lesquels la demanderesse a découvert que, de façon surprenante, ils inhibaient le métabolisme enzymatique de l'acide arachidonique provoqué par la cyclo-oxygénase et les lipoxygénases. Ce résultat est particulièrement inattendu en raison du blocage de la fonction acide sous la forme des amides définis ci-dessous. Elle a constaté, par ailleurs, que ces composés présentent une biodisponibilité différente de celle des acides correspondants, leur conférant des propriétés thérapeutiques améliorées et en particulier une activité particulièrement remarquable vis-à-vis de l'inhibition du métabolisme de l'acide arachidonique et notamment au niveau des lipoxygénases à l'origine de la formation des leucotriènes et des acides hydroxylés qui jouent un rôle important dans le processus inflammatoire.

L'invention a donc pour objet un nouveau procédé de préparation de cet acide eicosatétraynoïque-5,8,11,14, caractérisé par le fait que l'on traite l'heptyne-1 répondant à la formule :

$C_5H_{11}-C{\equiv}C-H$ (1)

avec une base forte pour former l'acétylure correspondant qui est amené à réagir avec le dihalogéno-1,4 butyne-2 de formule :

$XCH_2-C{\equiv}C-CH_2X$ (2)

où X représente un halogène, pour obtenir l'halogéno-1 undécadiyne-2,5 de formule :

$C_5H_{11}-C{\equiv}C-CH_2-C{\equiv}C-CH_2X$ (3)

que l'on amène à réagir avec le dianion de l'alcool propargylique, le produit résultant étant bromé par l'intermédiaire du tribromure de phosphore pour obtenir le bromo-1 tétradécatriyne-2,5,8 de formule :

$C_5H_{11}-C{\equiv}C-CH_2-C{\equiv}C-CH_2-C{\equiv}C-CH_2-Br$ (6)

qui est mis à réagir avec le dianion de l'acide hexyne-5 carboxylique pour obtenir l'acide eicosatétraynoïque.

Ce procédé consiste, dans un premier temps, à traiter l'heptyne-1 (1) par un dihalogéno-1,4 butyne (2). Ces deux réactifs (1) et (2) sont connus en euxmêmes. L'acétylure de l'heptyne (1) est en gènėral formé par échange avec un organomagnésien d'alkyle tel que, de préférence, le bromure d'éthylmagnésium.

Cet acétylure de l'heptyne est mis en réaction avec le dihalogénure (2) que l'on fait réagir en excès. On obtient, de cette façon, l'halogéno-1 undécadiyne-2,5 (3) avec un bon rendement.

L'intérêt principal de ce procédé est donc de passer en une étape d'une chaîne à 7 atomes de carbone à une chaîne à 11 atomes de carbone ayant en position-2 une triple liaison et en position-1 l'atome d'halogène.

Les synthèses ce cet halogénure décrites jusqu'à présent consistaient en des réactions d'allongement de chaîne à l'aide de l'alcool propargylique. L'alcool ainsi synthétisé était transformé en halogénure par action d'un trihalogénure de phosphore, ce qui nuisait au rendement du procédé.

Le passage de l'halogénure ayant 11 atomes de carbone de formule (3) à l'hydroxy-1 tétradécatriyne-2,5,8 (5) est réalisé par action du dianion de l'alcool propargylique (4). Cet alcool de formule (5) est à son tour transformé en bromo-1 tétradécatriyne-2,5,8 de formule (6), par action du tribromure de phosphore.

Ces deux dernières étapes sont décrites dans le brevet US-A-3.033.884.

L'acide eicosatétraynoïque-5,8,11,14 est obtenu en faisant réagir le dianion de l'acice hexyne-5 carboxylique suivant un procédé connu, sur le bromo-1 tétradécatriyne-2,5,8 de formule (6). La synthèse de cet acide hexyne-5 carboxylique est décrite en particulier dans la demande de brevet européen EP-A-86.109.150.

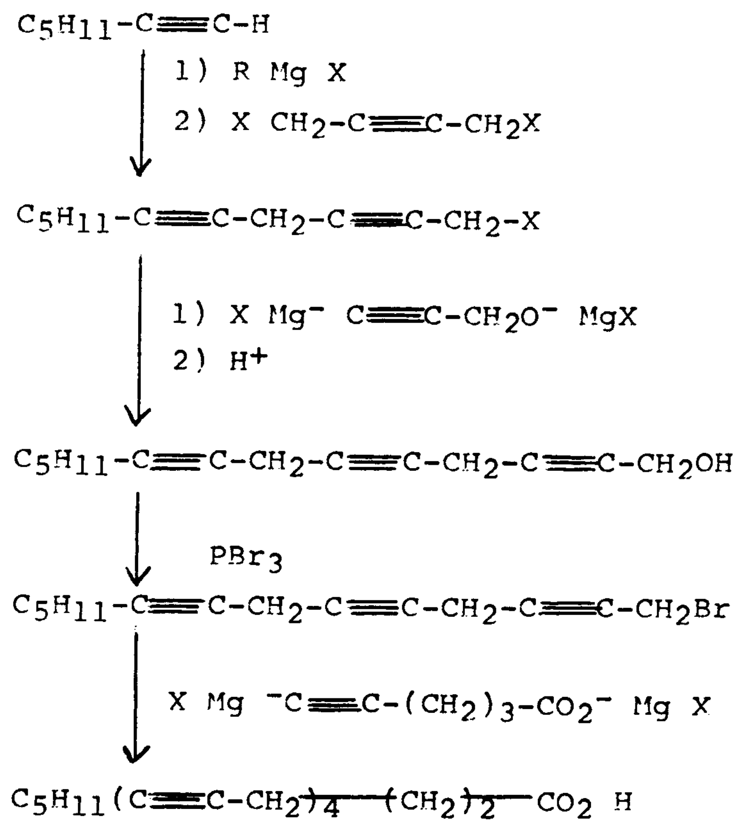


(1) (2) (3) (4) (5) (6) (7) (8)

SCHEMA-A-

où X représente un halogène.

Les amides évoqués ci-dessus sont alors obtenus en faisant réagir une forme activée de l'acide eicosatétraynoïque-5,8,11,14 sur une amine, dans un solvant organique.

Cette forme activée de l'acide peut être, soit un chlorure d'acide, un anhydride, ou encore l'intermédiaire formé par addition à une solution d'acide de carbonyl diimidazole (C.D.I.).

Cette dernière réaction est conduite de préférence en milieu solvant tel que le diméthylformamide ou encore dans un solvant chloré comme le dichlorométhane ou le dichloro-1,2 éthane. Cette réaction est illustrée par le schéma réactionnel B suivant :

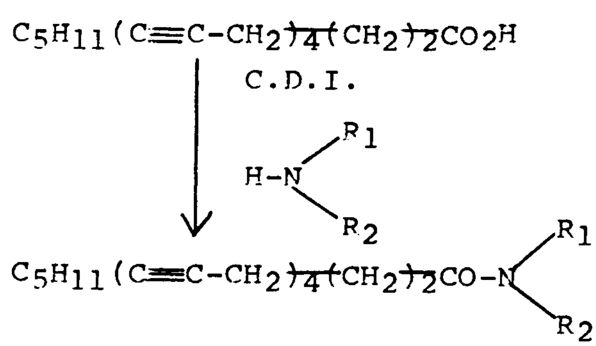


(I)

SCHEMA-B-

Dans la formule (I), $R_1$ et $R_2$ peuvent être identiques ou différents et correspondent à un atome d'hydrogène, un radical alkyle inférieur en $C_1$-$C_8$, linéaire ou ramifié, substitué au moins par un groupement hydroxyle. Ce radical alkyle inférieur en $C_1$-$C_8$ peut être interrompu par un ou plusieurs hétéroatomes

choisis parmi l'oxygène, l'azote ou le soufre;

$R_1$ et $R_2$ ne désignent pas simultanément un atome d'hydrogène;

$R_1$ et $R_2$ peuvent également former avec l'atome d'azote auquel ils sont rattachés, un hétérocycle comportant comme hétéroatome supplémentaire l'azote, l'oxygène ou le soufre, cet hétérocycle étant éventuellement substitué par un groupement alkyle ou hydroxyalkyle, les groupements alkyle ayant de préférence 1 à 8 atomes de carbone; le groupement amino peut également provenir d'un sucre et leurs sels d'acides minéraux ou organiques.

Les exemples suivants sont destinés à illustrer l'invention sans pour autant présenter un caractère limitatif.

## EXEMPLE

### Préparation de l'acide eicosatétraynoïque-5,8,11,14

#### a) Synthèse du chloro-1 undécadiyne-2,5

Dans un ballon équipé d'une arrivée d'argon, d'un réfrigérant et d'une ampoule à brome, introduire 4,17 g (0,17 mole) de magnésium. Recouvrir le magnésium de THF, ajouter quelques gouttes de bromure d'éthyle, amorcer la réaction par chauffage ou par introduction d'un cristal d'iode. Maintenir la réaction au reflux du THF par addition, au goutte à goutte, de 14 $cm^3$ de bromure d'éthyle en solution dans le THF, puis chauffer à 70-80 °C jusqu'à disparition totale du magnésium.

Ajouter 15 g d'heptyne (0,156 mole) en suivant le dégagement d'éthane et en maintenant la température à 70 °C. La réaction est très exothermique. Introduire 1,1 g de cyanure de cuivre et chauffer à 70 °C pendant une heure.

Refroidir le milieu réactionnel à 50 °C et ajouter rapidement 43 $cm^3$ (0,44 mole-2,8 équivalents) de dichlorobutyne. La réaction étant très exothermique, maintenir la température inférieure à 70 °C au cours de l'addition à l'aide d'un bain eau-glace puis chauffer à 70 °C pendant environ 2 heures.

En C.P.V., on constate la disparition totale de l'heptyne.

Verser le milieu réactionnel sur une solution glacée (500 $cm^3$) de chlorure d'ammonium et extraire à l'éther.

Laver la phase organique, sécher sur sulfate de magnésium et concentrer sous pression réduite.

Le produit attendu est purifié par distillation.

L'excès de dichlorobutyne est éliminé par distillation sous trompe à eau et le chloro-1 undécadiyne-2,5 est distillé sous vide de la pompe à palette.

Le produit attendu distille à 95-105 °C sous 0,01 mm Hg. Il est obtenu avec un rendement de 58%.

Le spectre $^1H$ RMN (80 MHz) est en accord avec la structure attendue.

#### b) Synthèse de l'hydroxy-1 tétradécatriyne-2,5,8

Dans un ballon muni d'une arrivée d'azote et d'une ampoule à brome, introduire 4,8 g de magnésium (0,197 mole). Recouvrir le magnésium de THF, ajouter quelques gouttes de bromure d'éthyle et amorcer la réaction par chauffage ou addition d'un cristal d'iode. Maintenir la réaction au reflux du THF par addition, au goutte à goutte, de 15 $cm^3$ de bromure d'éthyle (0,203 mole) en solution dans du THF, puis chauffer à 70-80 °C jusqu'à disparition totale du magnésium.

Refroidir le milieu réactionnel à 0 °C et ajouter au goutte à goutte, 6,2 $cm^3$ (0,105 mole) d'alcool propargylique distillé et conserve sur tamis moléculaire.

Maintenir la température à 0 °C pendant 30 minutes, puis chauffer à 70 °C pendant une heure pour terminer l'échange.

Refroidir le milieu réactionnel à 0 °C, ajouter 0,4 g de cyanure de cuivre puis chauffer à 40-45 °C pendant environ une heure.

Ajouter une solution de 12 g (0,066 mole) de chloro-1 undécadiyne-2,5 dans du THF et porter pendant environ 20 heures au reflux du THF.

La réaction est suivie en CCM. A la fin du temps de chauffage, il reste des traces de chloro-1 undécadiyne-2,5 de départ.

Verser le milieu réactionnel sur 600 $cm^3$ d'une solution de chlorure d'ammonium. Extraire à l'acétate d'éthyle, laver les phases organiques et sécher sur sulfate de magnésium. Par concentration sous pression réduite, on obtient une huile brune.

Reprendre cette huile dans de l'heptane chaud.

Par cristallisation au congélateur, on obtient 6,8 g (Rendement : 51%) d'une poudre blanche fondant à 26-27 °C.

Le spectre $^1H$ RMN est conforme à la structure attendue.

c) Synthèse du bromo-1 tétradécatriyne-2,5,8

Dans un ballon muni d'une arrivée d'argon, introduire 6 g d'hydroxy-1 tétradécatriyne-2,5,8 préparé précédemment, en solution dans 30 $cm^3$ d'éther éthylique. Ajouter 0,05 $cm^3$ de pyridine et additionner goutte à goutte 1 $cm^3$ de tribromure de phosphore puis porter au reflux pendant 3 heures.

Après avoir vérifié en CCM la fin de la réaction, verser le milieu réactionnel sur de l'eau glacée et extraire le produit attendu à l'éther. Laver les phases organiques avec une solution diluée de carbonate de sodium puis avec de l'eau. Sécher sur sulfate de magnésium et concentrer sous pression réduite.

On récupère 8 g d'une huile brune dont le spectre $^1H$ RMN (80 MHz) correspond à la structure attendue et qui sera utilisée telle quelle pour la suite des réactions.

d) Synthèse de l'acide eicosatétraynoïque-5,8,11,14

Dans un ballon muni d'une arrivée d'argon, d'un réfrigérant et d'une ampoule à brome, introduire 3,8 g (0,156 mole) de magnésium.

Recouvrir le magnésium de THF et réaliser le Grignard en ajoutant 13 $cm^3$ (0,172 mole) de bromure d'éthyle.

Maintenir le chauffage jusqu'à disparition totale du magnésium.

Refroidir le milieu réactionnel à 0 °C et introduire goutte à goutte 7,7 g d'acide hexyne-5 carboxylique en solution dans le THF.

Laisser remonter la température à 25 °C pendant 2 heures. Ajouter 0,6 g de cyanure de cuivre, maintenir l'agitation pendant 2 heures à température ambiante, puis ajouter 8 g (0,03 mole) de bromo-1 tétradécatriyne-2,5,8 obtenu précédemment en solution dans le THF.

Chauffer pendant environ 20 heures au reflux du THF. En CCM, on ne constate plus d'évolution de la réaction.

Verser le milieu réactionnel sur une solution diluée d'acide chlorhydrique dans de la glace. Extraire à l'acétate d'éthyle.

Laver les phases organiques avec un solution d'hydrogéno-carbonate de sodium pour éliminer l'excès d'acide hexyne-5 carboxylique. Laver à nouveau à l'eau puis sécher la phase organique.

Concentrer sous pression réduite puis recristalliser le produit brut ainsi obtenu dans un minimum de méthanol.

On récupère 1,5 g de cristaux légèrement crème dont le point de fusion est de 78-80 °C et qui semblent hygroscopiques.

Les spectres $^1H$ RMN et IR sont conformes avec la structure attendue.

Analyse élémentaire : $C_{20}H_{24}O_2$

| | | C | H | O |
|---|---|---|---|---|
| Valeur théorique | : | 81,04 | 8,16 | 10,72 |
| Valeur trouvée | : | 77,96 | 8,25 | 13,73 |
| Valeur théorique avec 2/3 $H_2O$ | : | 77,95 | 8,21 | 13,85 |

EXEMPLE DE PREPARATION 1

Synthèse du N-(hydroxy-2 éthyloxyéthyl) eicosatétraynamide-5,8,11,14

A une solution de 1,5 g d'acide eicosatétraynoïque-5,8,11,14 (5 mmoles) dans 30 $cm^3$ de DMF anhydre, dégazée à l'argon, ajouter 1,1 g de carbonyldiimidazole et chauffer à 50 °C pendant 1 heure 30 minutes.

Refroidir la solution à 0 °c, puis additionner 1,05 g (0,01 mole) d'hydroxy-2 éthyloxyéthylamine en solution dans 10 $cm^3$ de DMF.

Laisser revenir à température ambiante pendant 2 heures puis, après avoir vérifié en CCM la disparition de l'acide de départ, verser le milieu réactionnel sur une solution diluée de chlorure d'ammonium, extraire à l'acétate d'éthyle. Laver à l'eau les phases organiques. Sécher et concentrer sous pression réduite.

Cristalliser le brut de réaction dans l'heptane.

Par recristallisation dans l'éther diisopropylique, on obtient 1,1 g d'une poudre blanche dont le point de fusion est de 68-69 °C.

Les spectres $^{13}C$ RMN et IR sont conformes à la structure attendue.

Analyse élémentaire : $C_{24}H_{33}NO_3$

| | | C | H | N | O |
|---|---|---|---|---|---|
| Valeur théorique | : | 75,16 | 8,67 | 3,65 | 12,51 |
| Valeur trouvée | : | 74,60 | 8,73 | 3,81 | 13,23 |

EXEMPLE DE PREPARATION 2

Synthèse de l'(hydroxy-2 éthyl)-4'pipérazino eicosatétraynamide-5,8,11,14

A une solution de 1,5 g d'acide eicosatétraynoïque-5,8,11,14 (5 mmoles) dans 30 $cm^3$ de DMF anhydre, dégazée à l'argon, ajouter 1,1 g de carbonyldiimidazole et chauffer à 50 °C pendant 1 heure 30 minutes.

Refroidir la solution à 0 °C, puis additionner 1,3 g d'hydroxy-2 éthylpipérazine (0,01 mole) en solution dans 10 $cm^3$ de DMF.

Laisser revenir à température ambiante pendant 2 heures, puis après avoir vérifié la disparition de l'acide de départ en CCM, verser le milieu réactionnel sur une solution diluée de chlorure d'ammonium. Extraire à l'éther diisopropylique, laver les phases organiques abondamment à l'eau pour éliminer l'excès d'hydroxy-2 éthylpipérazine. Sécher et concentrer sous pression réduite.

On récupère 1 g d'une huile brune dont les spectres $^{13}C$ RMN et IR sont conformes à la structure attendue.

| | | C | H | N | O |
|---|---|---|---|---|---|
| Valeur théorique | : | 77,96 | 7,04 | 6,99 | 7,99 |
| Valeur trouvée | : | 74,65 | 8,54 | 6,94 | 9,99 |
| Valeur théorique avec 3/4 $H_2O$ | : | 75,18 | 7,20 | 6,78 | 10,64 |

EXEMPLE DE PREPARATION 3

Synthèse du N-(hydroxy-2 éthyl)eicosatétraynamide-5,8,11,14

A une solution de 500 mg d'acide eicosatétraynoïque-5,8,11,14 (1,7 mmole) dans 20 $cm^3$ de dichloro-1,2 éthane anhydre, dégazée à l'argon, ajouter 360 mg de carbonyldiimidazole en maintenant la solution à 15 °C pendant environ 1 heure.

Refroidir la solution à 0 °C, puis additionner 200 mg d'éthanolamine en solution dans du dichloro-1,2 éthane.

Abandonner pendant une nuit à température ambiante, puis après avoir vérifié en CCM la disparition de l'acide de départ, verser le milieu réactionnel sur une solution diluée d'acide chlorhydrique.

Extraire au dichlorométhane, laver les phases organiques abondamment à l'eau pour éliminer l'excès d'éthanolamine. Sécher et concentrer sous pression réduite.

On récupère 250 mg d'une poudre blanche qui est recristallisée dans l'acétonitrile et dont le point de fusion est de 93-94 °C.

Les spectres $^{1}H$ RMN (80 MHz) et IR correspondent à la structure attendue.

EXEMPLE DE PREPARATION 4

Synthèse du N-(dihydroxy-2,3 propyl)eicosatétraynamide-5,8,11,14

A une solution de 500 mg d'acide eicosatétraynoïque-5,8,11,14 (1,7 mmole) dans 20 $cm^3$ de dichloro-1,2 éthane anhydre, dégazée à l'argon, ajouter 360 mg de carbonyldiimidazole en maintenant la solution à 15 ° C pendant environ une heure.

Refroidir la solution à 0 ° C, puis additionner 310 mg d'amino-3 propanediol-1,2 en solution dans du dichloro-1,2 éthane. Abandonner une nuit à température ambiante, puis après avoir vérifié en CCM la disparition de l'acide de départ, verser le milieu réactionnel sur une solution diluée d'acide chlorhydrique.

Extraire au dichlorométhane, laver les phases organiques abondamment à l'eau pour éliminer l'excès d'amino-3 propanediol-1,2. Sécher et concentrer sous pression réduite.

On récupère 200 mg d'une poudre blanche qui est purifiée par chromatographie sur gel de silice (éluant : chloroforme/acétate d'éthyle/méthanol) et dont le point de fusion est de 95-96 ° C.

Les spectres $^1$H RMN (80 MHz) et IR sont conformes à la structure attendue.

**Revendications**

1. Procédé de préparation de l'acide eicosatétraynoïque, caractérisé par le fait que l'on traite l'heptyne-1 répondant à la formule :

   $C_5H_{11}$-C≡C-H (1)

   avec une base forte pour former l'acétylure correspondant qui est amené à réagir avec le dihalogéno-1,4 butyne-2 de formule :

   $XCH_2$-C≡C-$CH_2X$ (2)

   où X représente un halogène,
   pour obtenir l'halogéno-1 undécadiyne-2,5 de formule :

   $C_5H_{11}$-C≡C-$CH_2$-C≡C-$CH_2X$ (3)

   que l'on amène à réagir avec le dianion de l'alcool propargylique, le produit résultant étant bromé par l'intermédiaire du tribromure de phosphore pour obtenir le bromo-1 tétradécatriyne-2,5,8 de formule :

   $C_5H_{11}$-C≡C-$CH_2$-C≡C-$CH_2$-C≡C-$CH_2$-Br (6)

   qui est mis à réagir avec le dianion de l'acide hexyne-5 carboxylique pour obtenir l'acide eicosatétraynoïque.

2. Procédé selon la revendication 1, caractérisé en ce que, pour obtenir l'halogéno-1 undécadiyne-2,5, on traite l'heptyne par un organomagnésium d'alkyle pour former l'acétylure de l'heptyne qui est mis en réaction avec le dihalogéno-1,4 butyne-2.

3. Procédé selon la revendication 2, caractérisé en ce que l'organomagnésium est le bromure d'éthylmagnésium.

4. Procédé selon la revendication 2 ou 3, caractérisé en ce que le dihalogéno-1,4 butyne-2 est employé en excès.

**Claims**

1. Process for the preparation of eicosatetraynoic acid, characterized in that 1-heptyne corresponding to the formula:

   $C_5H_{11}$-C≡C-H (1)

is treated with a strong base in order to form the corresponding acetylide, which is made to react with the 1,4-dihalo-2-butyne of formula:

$XCH_2$-C≡C-$CH_2X$ (2)

where X represents a halogen,
in order to obtain the 1-halo-2,5-undecadiyne of formula:

$C_5H_{11}$-C≡C-$CH_2$-C≡C-$CH_2X$ (3)

which is made to react with the dianion of propargyl alcohol, the resultant product being brominated using phosphorus tribromide in order to obtain 1-bromo-2,5,8-tetradecatriyne of formula:

$C_5H_{11}$-C≡C-$CH_2$-C≡C-$CH_2$-C≡C-$CH_2$-Br (6)

which is reacted with the dianion of 5-hexynecarboxylic acid in order to obtain eicosatetraynoic acid.

**2.** Process according to Claim 1, characterized in that, in order to obtain the 1-halo-2,5-undecadiyne, heptyne is treated with an alkyl organomagnesium in order to form heptyne acetylide which is reacted with the 1,4-dihalo-2-butyne.

**3.** Process according to Claim 2, characterized in that the organomagnesium is ethylmagnesium bromide.

**4.** Process according to Claim 2 or 3, characterized in that the 1,4-dihalo-2-butyne is used in excess.

**Patentansprüche**

**1.** Verfahren zur Herstellung von Eicosatetrainsäure, dadurch **gekennzeichnet,** daß man 1-Heptin der Formel:

$C_5H_{11}$-C≡C-H (1)

mit einer starken Base behandelt, um das entsprechende Acetylid zu bilden, das mit 1,4-Dihalogen-2-butin der Formel zur Reaktion gebracht wird:

$XCH_2$-C≡C-$CH_2X$ (2)

worin X ein Halogen darstellt,
um 1-Halogen-2,5-undecadiin der Formel zu erhalten:

$C_5H_{11}$≡C-$CH_2$-C≡C-$CH_2X$ (3)

das man mit dem Dianion von Propargylalkohol zur Reaktion bringt, worauf das entstandene Produkt über die Zwischenstufe des Phosphortribromids bromiert wird, um 1-Brom-2,5,8-tetradecatriin der Formel zu erhalten:

$C_5H_{11}$-C≡C-$CH_2$-C≡C-$CH_2$-C≡C-$CH_2$-Br (6)

welches mit dem Dianion von 5-Hexincarboxylsäue zur Reaktion gebracht wird, um die Eicosatetrainsäure zu erhalten.

**2.** Verfahren gemäß Anspruch 1,
dadurch **gekennzeichnet,** daß man zum Erhalt da 1-Halogen-2,5-undecadiin Heptin mit einer Organomagnesium-Alkylverbindung behandelt, um das Heptinacetylid zu bilden, das mit 1,4-Dihalogen-2-butin umgesetzt wird.

**3.** Verfahren gemäß Anspruch 2,
dadurch **gekennzeichnet**, daß die Organomagnesium-Verbindung Ethylmagnesiumbromid ist.

4. Verfahren gemäß Anspruch 2 oder 3, dadurch **gekennzeichnet**, daß das 1,4-Dihalogen-2-butin im Überschuß eingesetzt wird.